# FASCICULE DE BREVET EUROPEEN

(11) **EP 2 214 704 B1**
(45) Date de publication et mention de la délivrance du brevet: **27.11.2013**
(21) Numéro de dépôt: 08865695.4
(22) Date de dépôt: 03.12.2008
(51) Int. Cl.: A61K 39/35, A61K 39/36, A61P 37/08, A61B 17/20, A61K 9/70, A61K 9/14, A61F 13/00

(54) **METHODE DE DESENSIBILISATION AUX ALLERGENES**
ALLERGENDESENSIBILISIERUNGSVERFAHREN
ALLERGEN DESENSITIZATION METHOD

(30) Priorité: 03.12.2007 FR 0759503; 29.07.2008 US 84305
(43) Date de publication de la demande: 11.08.2010
(73) Titulaire: DBV Technologies, 75014 Paris (FR)
(72) Inventeur: DUPONT, Christophe, 92140 Clamart (FR); BENHAMOU, Pierre-Henri, 75015 Paris (FR); DUPONT, Bertrand, 13100 Aix en Provence (FR)
(74) Mandataire: Becker, Philippe
(86) Numéro de dépôt international: PCT/FR2008/052199
(87) Numéro de publication internationale: WO 2009/080934

(56) Documents cités:
- EP-A- 1 031 346
- WO-A-02/071950
- WO-A-02/093998
- WO-A-2007/122226
- DIOSZEGHY VINCENT ET AL: "Epicutaneous immunotherapy results in rapid allergen uptake by dendritic cells through intact skin and downregulates the allergen-specific response in sensitized mice.", JOURNAL OF IMMUNOLOGY (BALTIMORE, MD. : 1950) 15 MAY 2011 LNKD- PUBMED:21490160, vol. 186, no. 10, 15 May 2011 (2011-05-15) , pages 5629-5637, ISSN: 1550-6606

## Description

La présente demande concerne une méthode pour la désensibilisation de patients allergiques. Elle concerne plus précisément une méthode de désensibilisation épicutanée, applicable à tout type d'allergènes et de patients. La méthode de l'invention est essentiellement non invasive et ne requiert pas l'utilisation d'adjuvants. En outre, elle peut être aisément mise en oeuvre et suivie par le patient lui-même.

L'allergie occupe une place de plus en plus importante en pratique médicale quotidienne. Il s'agit d'un phénomène mondial de santé publique classé quatrième fléau mondial par l'OMS. En France, un Français sur trois est allergique et l'allergie aussi bien respiratoire qu'alimentaire touche une partie croissante de la population d'adultes et d'enfants.

La désensibilisation est la technique selon laquelle l'administration de quantités minimes d'allergènes peut permettre d'éteindre plus ou moins le phénomène allergique. Depuis le début des années 2000, elle est reconnue comme la seule méthode de traitement de fond de l'allergie par l'OMS.

Il est maintenant établi que la désensibilisation affecte la réponse immunitaire vis-à-vis des allergènes dès ses premières étapes (Tij *et al*., 2004), ce qui signifie qu'elle réduit non seulement les symptômes liés à la réaction allergique à court terme mais également qu'elle modifie l'histoire naturelle de la « maladie » allergique et qu'elle prévient à la fois l'apparition d'allergie à de nouveaux allergènes mais également l'évolution de la symptomatologie vers des manifestations cliniques plus sévères, comme par exemple la transformation d'une rhinite allergique en asthme.

L'immunothérapie s'est avérée efficace chez des patients atteints d'allergie IgE dépendante sévère, sensibilisés à un nombre restreint d'allergènes.

Bien que les mécanismes d'action des traitements par immunothérapie ne soient pas encore bien connus, celle-ci pourrait agir par :
- une augmentation des IgG et en particulier des fractions IgG4, anticorps bloquant in vitro les effets biologiques des IgE, même si l'importance de ces effets in vivo doit encore être évaluée,
- une altération de la balance TH1/TH2, favorisant la réponse TH1,
- la production de cellules T produisant l'interleukine 10 (IL-10). Celle-ci possède de nombreuses propriétés anti-allergiques contre les mastocytes, certains lymphocytes T et les éosinophiles et favorise également la production d'IgG4.

L'immunothérapie par voie sous-cutanée représente pour la plupart des allergologues la voie de référence et elle est encore largement utilisée. Bien que coûteuse et nécessitant l'intervention d'un médecin spécialiste à chaque injection, elle est aujourd'hui encore considérée comme la voie de référence de la désensibilisation chez l'enfant (Pajno *et al*., 2005). Elle n'est cependant pas sans risques, puisque la fréquence des décès est estimée à 1 pour 2,5 millions d'injections avec une moyenne de 3,4 décès par an.

Certains allergènes à haut risque de réaction anaphylactiques ne sont pas utilisés dans le cadre de cures de désensibilisation sous-cutanée. Il en est ainsi de l'arachide et de la plus grande partie des allergènes alimentaires.

L'immunothérapie sub-linguale est maintenant considérée par l'OMS comme une alternative satisfaisante à l'immunothérapie par voie sous-cutanée. Elle est en cours d'évaluation dans de nombreux pays. Les premières préparations commerciales de comprimés à administrer sous la langue, destinés à un large public sont en vente dans certains pays européens et en pré- commercialisation aux Etats-Unis.

Beaucoup mieux supportée par les patients, l'immunothérapie sub-linguale est, pour beaucoup d'auteurs, d'un meilleur rapport coût-efficacité que les autres voies d'administration puisqu'elle ne nécessite pas l'intervention d'un médecin et peut être auto-administrée par les patients qui déclarent la préférer aux autres voies (Pajno et al., 2005).

Les réactions adverses sont rares lors des traitements par voie sub-linguale, estimés de 0,1 à 0,2 réactions pour 1000 doses administrées. Les réactions sont principalement mineures, affectant la cavité buccale ou la zone sub-linguale ou la sphère gastro-intestinale (La Rosa *et al.*, 1999).

En terme d'efficacité, la voie sub-linguale est cependant considérée par certains comme moins efficace que la voie sous-cutanée, elle nécessite l'utilisation d'une quantité supérieure d'allergène et ne peut être autorisée avec tous les allergènes même si des essais de désensibilisation par cette voie à l'arachide et au lait de vache sont en cours. La sécurité d'emploi de cette technique avec les allergènes alimentaires reste à évaluer.

L'immunothérapie intra-nasale s'est révélée efficace dans 17 sur 18 études contrôlées. Il s'agit donc d'une voie efficace et sûre tout au moins au cours de la rhinite allergique. Cependant, elle est généralement mal tolérée par les patients, ce qui limite de plus en plus son utilisation en clinique. Dans une étude comparant les différentes voies de désensibilisation, les traitements par voie nasale étaient interrompus prématurément dans près de 50 % des cas avant un an, alors que les arrêts prématurés ne concernaient que moins de 10 % des enfants traités par voie sous-cutanée ou sub-linguale (Pajno et al., 2005).

Une alternative avantageuse à ces méthodes de désensibilisation résiderait dans la possibilité d'effectuer l'immunothérapie par la voie épicutanée, c'est-à-dire par l'application répétée d'un allergène sur la peau, conduisant typiquement à la diffusion de l'allergène dans les couches superficielles de la peau, généralement sans passage transcutané significatif.

Il a été démontré de longue date chez l'animal que l'application répétée d'un allergène sur la peau est susceptible de provoquer une sensibilisation de l'animal audit allergène (Golovanoff, 1926). La réaction obtenue est de type systémique et atteint l'organisme dans son ensemble et donc bien au-delà de la simple sphère cutanée.

Plus récemment, des modèles de souris sensibilisés au lait de vache par voie épicutanée ont été développés (Chang). Il a été montré que dans ces cas, la réponse immunitaire suscitée fait intervenir différentes populations cellulaires et aboutit à l'activation de lymphocytes TH2, qui favorisent la production d'anticorps de type IgE spécifiques de l'allergène.

Certains travaux ont permis d'établir que si la réponse spontanée de la peau dans ces conditions de stimulation était bien de type TH2, il était possible de modifier le profil immunitaire de la réponse en soumettant la peau à différents stimuli :
- Agressions par agents physiques tels que les rayons ultra-violets
- Agression mécanique de la peau par stripping répétés modifiant la constitution de la couche cornée
- Agression par des agents microbiens tels que la toxine cholérique
- Modification de la perméabilité cutanée.

Le type de réponse immunitaire au contact d'un allergène peut être modifié par ces différents agents et aboutit à l'activation préférentielle des lymphocytes TH1 favorisant la production d'anticorps de type IgG4. La voie épicutanée a été utilisée par les allergologues au cours des années 50. Elle consistait à pratiquer une scarification sur la peau à travers une goutte d'allergène plusieurs fois par semaine (Pautrizel *et al*., 1957). Pratiquée par un allergologue expérimenté, cette technique offrait efficacité et sécurité. Cependant, très artisanale et peu standardisée, elle est restée peu diffusée. Finalement, la pénibilité engendrée par les scarifications répétées souvent très étendues et mutilantes est sans nul doute la raison pour laquelle cette méthode est aujourd'hui abandonnée.

La demande de brevet EP 1031346 propose un vaccin contenant un dispositif transdermal et un antigène ou un allergène afin d'obtenir une désensibilisation. Les méthodes décrites dans ce document utilisent des composés induisant l'activité de cytokines (e. g., adjuvants) et ne visent pas une réaction inflammatoire de type tolérogène permettant de désensibiliser de manière efficace les sujets allergiques. La demande WO 2007/122226 mentionne l'utilisation d'un dispositif cutané pour la désensibilisation d'un sujet allergique, mais ne mentionne pas l'induction et l'entretien d'une réaction inflammatoire.

La présente invention réside dans la mise au point d'une méthode de désensibilisation épicutanée efficace et qui permet de surmonter les inconvénients et limites des techniques antérieures.

Plus précisément, l'invention repose en partie sur la mise en évidence que la réponse inflammatoire spécifique provoquée par l'application cutanée d'allergènes influence grandement et participe à la réaction des cellules immunitaires dans le sens de la tolérance.

L'invention consiste à appliquer un allergène de façon répétée et prolongée sur la peau de façon à provoquer une réaction inflammatoire dont le profil, orienté par la durée et la répétition des applications est de type « tolérogène ». Ainsi, l'invention vise non seulement à provoquer la réaction inflammatoire mais également à l'orienter.

La présente demande montre que le contrôle de cette réaction inflammatoire conditionne le degré de tolérance obtenu et représente un élément essentiel du processus de désensibilisation épicutanée. L'invention montre que la provocation répétée de cette réaction inflammatoire spécifique est importante pour inverser progressivement la réponse immunitaire dans le sens de la tolérance, et qu'elle rend possible la désensibilisation épicutanée, même en l'absence d'adjuvant ou de prétraitement de la peau.

L'invention propose ainsi, pour la première fois, une méthode de désensibilisation cutanée comprenant l'application cutanée répétée d'un allergène sur la peau du sujet dans des conditions permettant d'entretenir, au cours du traitement, l'inflammation générée par l'application de l'allergène sur la peau, comme défini dans les revendications. Avantageusement, la méthode comprend au moins une étape de suivi et/ou d'évaluation du degré de l'inflammation générée par l'application de l'allergène sur la peau, la provocation, l'entretien et/ou le contrôle de cette réaction permettant de diriger la réaction de l'organisme à l'allergène dans le sens de la tolérance.

Un objet de l'invention réside ainsi dans l'utilisation d'un allergène pour la préparation d'une composition pour la désensibilisation épicutanée d'un sujet allergique au dit allergène, par applications répétées de l'allergène sans adjuvant sur la peau du sujet au moyen d'un dispositif cutané non perforant assurant un contact entre l'allergène et la peau dans des conditions de dose, ou de fréquence ou de temps d'application, ou de forme de l'allergène permettant de provoquer une réaction inflammatoire cutanée sur la zone d'application de l'allergène, et d'entretenir ladite réaction inflammatoire au cours du traitement,. L'invention montre en effet que l'entretien de cette réaction inflammatoire favorise une réponse de type tolérogène du système immunitaire vis-à-vis de l'allergène.

L'invention décrit aussi l'utilisation d'un allergène pour la préparation d'une composition pour la désensibilisation épicutanée d'un sujet allergique au dit allergène, par applications répétées de l'allergène sur la peau du sujet, caractérisée en ce qu'elle comprend, au cours du traitement, de préférence lors de chaque application de l'allergène, une étape de contrôle et/ou d'entretien de la réaction inflammatoire générée par l'application de l'allergène sur la peau.

L'invention décrit en outre l'utilisation d'un allergène pour la préparation d'une composition pour la désensibilisation épicutanée d'un sujet allergique au dit allergène, par applications répétées de l'allergène sur la peau du sujet, caractérisée en ce qu'elle comprend, au cours du traitement, le contrôle et l'entretien de la réaction inflammatoire générée par l'application de l'allergène sur la peau.

Un autre objet de l'invention réside dans l'utilisation d'un allergène pour la préparation d'une composition pour modifier dans le sens de la tolérisation la réponse immunitaire d'un sujet allergique audit allergène, comprenant l'application répétée de l'allergène sur la peau du sujet dans des conditions permettant d'entretenir au cours du traitement une réaction inflammatoire.

L'invention concerne encore une méthode de désensibilisation épicutanée d'un sujet allergique à un allergène, par application répétée de l'allergène sur la peau du sujet, caractérisée en ce que l'allergène est appliqué sur la peau dans des conditions permettant d'entretenir au cours du traitement une réaction inflammatoire.

Comme il sera décrit en détails dans la suite de la demande, la réaction inflammatoire est avantageusement entretenue en contrôlant ou modulant au cours du traitement la dose ou la fréquence ou le temps d'application de l'allergène, et/ou sa nature.

Un autre objet de l'invention réside dans une méthode de désensibilisation épicutanée d'un sujet allergique à un allergène, caractérisée en ce qu'elle comprend au moins une étape d'évaluation, au cours du traitement, du degré de l'inflammation générée par l'application de l'allergène sur la peau. L'évaluation du degré de la réaction inflammatoire permet d'ajuster, si nécessaire, les conditions de la désensibilisation.

L'invention concerne encore une méthode de désensibilisation d'un sujet allergique à un allergène, par application répétée de l'allergène sur la peau du sujet, caractérisée en ce qu'elle comprend, à chaque application de l'allergène, une étape de contrôle de la réaction inflammatoire.

L'invention concerne encore une méthode de désensibilisation d'un sujet allergique à un allergène, par application répétée de l'allergène sur la peau du sujet, caractérisée en ce qu'elle comprend l'entretien, au cours du traitement, de la réaction inflammatoire générée par l'application de l'allergène sur la peau.

L'invention concerne également l'utilisation de la réaction inflammatoire générée par l'application d'un allergène sur la peau d'un patient comme marqueur pour adapter le traitement de désensibilisation épicutanée dudit patient audit allergène.

Comme il sera décrit dans la suite de la demande, l'invention est applicable à tout type d'allergène et peut être mise en oeuvre avec tout dispositif d'application cutané. Elle permet une désensibilisation efficace sans qu'il soit nécessaire de traiter au préalable la peau du patient. Par ailleurs, elle ne requiert pas la co-administration de composés adjuvants.

L'utilisation d'allergène déposé sur la peau d'un sujet allergique est parfaitement sûre, comme l'a démontré l'expérience accumulée dans le domaine diagnostic avec les atopy patch test. Dans notre expérience en France avec le Diallertest^{®}, premier atopy patch test prêt à l'emploi commercialisé depuis 2004, aucun effet adverse n'a été rapporté, qu'il s'agisse d'une réaction locale sévère ou d'une réaction généralisée de type systémique.

Après l'application d'un allergène sur la peau, le système immunitaire peut soit ne pas réagir soit réagir par une réaction inflammatoire locale (cutanée) ou une réaction immunitaire systémique.

Dans un mode particulier de mise en oeuvre de l'invention, l'application d'un allergène sur la peau chez le sujet allergique audit allergène induit une réaction cutanée, en particulier de type inflammatoire, déclenchée par le contact de l'allergène avec les kératinocytes et cellules de Langerhans présents à la surface de la peau.

Au cours de l'immunothérapie épicutanée par la méthode de l'invention, cette réaction provoquée par l'application de l'allergène sur la peau d'un sujet allergique audit allergène, est entretenue et/ou contrôlée durant la cure. L'invention montre en effet que le maintien et le contrôle de cette réaction aboutissent à une orientation progressive de la réponse immunitaire dans le sens de la tolérance du sujet audit allergène. Cette réponse est matérialisée cliniquement par une diminution progressive ou une extinction complète de la réactivité cutanée et par une augmentation du seuil de tolérance audit allergène.

Le mécanisme énoncé ci-dessus s'applique à tout allergène, notamment aux allergènes agissant sur la sphère digestive (allergie alimentaire, comme l'ovalbumine, les arachides, les crustacées, etc.), pulmonaire (allergie respiratoire, comme par exemple le pollen) ou cutanée.

Ainsi, le principe sur lequel est basée la méthode de l'invention et qui est parfaitement illustré par les données expérimentales exposées ci après, est qu'il est possible d'induire, par voie cutanée, une tolérance d'un sujet préalablement sensibilisé à un allergène. Cet effet est obtenu en maintenant l'allergène sur la peau du sujet allergique audit allergène et ce faisant en provoquant, entretenant et contrôlant la réaction inflammatoire à l'allergène dans le but de diriger la réaction de l'organisme à l'allergène dans le sens de la tolérance.

Ainsi, la méthode d'immunothérapie épicutanée de l'invention est une méthode thérapeutique de l'allergie qui consiste à mettre en présence les cellules immunitaires de la peau (e.g. cellules de Langerhans, mastocytes, macrophages, leucocytes) avec un allergène chez un sujet allergique audit allergène et à provoquer par le biais de la réaction inflammatoire cellulaire spécifique, contrôlée et entretenue une conversion de la réponse immunitaire aboutissant à la tolérance dudit allergène par le patient allergique. Dans le procédé de l'invention, et contrairement à toutes les approches de désensibilisation antérieures, le processus de désensibilisation est suivi et guidé par la nature et le degré de la réaction inflammatoire du patient. Cette approche assure une désensibilisation efficace et ajustable aux besoins et aux caractéristiques de chaque patient, et ne nécessite pas l'emploi de composés adjuvants ni le traitement préalable perforant ou abrasif de la peau.

La méthode de l'invention comprend, de manière générale, l'application de façon répétée, prolongée et contrôlée d'un allergène sur la peau d'un sujet allergique audit allergène, dans des conditions (par exemple de dose et/ou de fréquence et/ou de durée d'application) permettant le maintien d'une réaction inflammatoire spécifique à l'allergène au cours du traitement. Dans un mode avantageux de mise en oeuvre, l'application est réalisée sans composé adjuvant et/ou sur peau saine.
La méthode de l'invention comprend, de manière générale, les phases suivantes :
a. de manière facultative, la sélection des patients et/ou de l'allergène (dose, nature, formulation, etc.) ;
b. l'application de l'allergène au patient ; et
c. le contrôle de la réaction inflammatoire ; les étapes b. et c. étant avantageusement répétées une ou plusieurs fois au cours du traitement, l'état de l'allergène (dose, nature, formulation, fréquence d'application, temps de pose, etc.) étant adapté au cours du traitement pour entretenir la réaction inflammatoire.
Dans la mesure où l'invention repose sur le contrôle de la réaction inflammatoire, il est important que les patients allergiques soumis au traitement présentent une telle réponse lors de la première application de l'allergène sur la peau. Pour cela, le patient est avantageusement soumis à une première (série) d'application de l'allergène, de manière à déterminer la préparation d'allergène donnant la réactivité la plus appropriée au début du traitement.
Dans ce but, le patient est avantageusement soumis à une première application de l'allergène, sous une forme et à une dose unique prédéfinies.
Si le sujet développe une réaction inflammatoire appropriée, le traitement peut être engagé sur la base de cette dose/forme d'allergène. Une réaction appropriée désigne par exemple une réaction d'inflammation visible à l'oeil nu, typiquement sous forme de lésions érythémateuses infiltrées pourvues ou non de papules ou prenant un aspect eczématiforme.
Si la réaction constatée est de forte intensité, comportant des vésicules voire une phlyctène, ou est largement diffusée dans les zones cutanées avoisinantes ou fortement prurigineuse, la concentration d'allergène pour le lancement de la cure sera diminuée, pour obtenir une réponse appropriée ou, en variante, la durée d'application de l'allergène sur la peau sera réduite.
Au contraire, lorsque la réaction constatée est de trop faible intensité, les doses d'allergène et/ou la durée d'application et/ou le nombre de dispositifs appliqués simultanément sera augmenté pour obtenir une réaction initiale significative telle que définie ci-dessus. Dans ce cas, il est également possible de tester d'autres formes de l'allergène, pour identifier des préparations donnant une meilleure réactivité du sujet. Ainsi en effet, l'allergène peut exister sous différentes formes (aliment complet, extrait protéique, peptides, produits recombinants ou de synthèse, etc.) pouvant provoquer une réponse inflammatoire différente chez le patient allergique. Cette première phase du traitement peut ainsi permettre de déterminer la forme de l'allergène la plus adaptée pour le début de la cure.
A cet égard, dans un mode de mise en oeuvre particulier, le patient est soumis au temps initial de la cure à une série de dispositifs contenant des concentrations et/ou formes variées de l'allergène. Ce premier traitement permet de déterminer la dose et/ou la forme optimale, patient par patient, servant de référence à initier la cure. Le cas échéant, l'utilisation de ces dispositifs à concentration et/ou forme variées pourra être répétée au cours de la cure de façon à adapter la dose/forme utilisée en cours de traitement, en fonction de l'évolution de la réactivité du sujet.
Lorsque la dose/forme initiale a été déterminée, le traitement d'immunothérapie épicutanée peut être engagé. Ce traitement comprend l'application répétée de l'allergène sur la peau. L'application s'effectue avantageusement à la partie supérieure du dos du sujet ou à la face interne des bras ou des avants bras. Elle peut également s'effectuer sur toute autre région du corps, à la condition préférentielle que l'on ait auparavant vérifié la réactivité locale à l'allergène. Il est entendu que, au cours du traitement, l'allergène peut être appliqué sur des régions différentes du corps. Les applications sont renouvelées à une fréquence variable, dépendant du protocole thérapeutique utilisé (un protocole typique comprend un temps initial d'application compris entre 6 et 72h environ). Ils peuvent être appliqués en continu ou de façon discontinue en respectant des périodes de durée variable entre les applications (qui peuvent aller de 1 jour à trois semaines, par exemple). L'application répétée et prolongée pendant plusieurs mois de l'allergène aboutit à la libération locale de médiateurs de l'inflammation et à l'afflux de cellules inflammatoires qui influencent la réaction des cellules immunitaires dans le sens de la tolérance. Un protocole typique comprend l'application répétée de l'allergène, pendant des périodes de 6 à 72 heures environ, espacées de 5 à 15j environ, sur une durée nécessaire pour désensibiliser le sujet, cette durée pouvant excéder plusieurs mois.
Pour assurer une désensibilisation appropriée, la présente invention démontre qu'il est important de contrôler et d'entretenir (par la modulation des doses et/ou des temps ou fréquence de pose de l'allergène, ou de sa forme), au cours du traitement, une réaction d'inflammation locale. La méthode de l'invention comprend ainsi une évaluation régulière du degré de l'inflammation générée par l'application répétée de l'allergène sur la peau. Ainsi, au cours de la cure, les doses d'allergène et/ou la durée et/ou le nombre de dispositifs utilisés simultanément sera(ont) adaptés en fonction du degré de la réactivité cutanée. Une diminution de l'intensité de la réaction cutanée au cours du traitement aboutit à une augmentation de la fréquence d'application et/ou du nombre de dispositifs appliqués simultanément et/ou de la dose de l'allergène, et/ou à une modification de sa forme. Au contraire, une augmentation de l'intensité de la réaction cutanée aboutit à une diminution de la fréquence d'application et/ou du nombre de dispositifs appliqués simultanément et/ou de la concentration de l'allergène dans ledit dispositif, et/ou de sa forme.
L'évaluation de l'intensité de la réaction inflammatoire, en particulier de la réaction cutanée, peut être effectuée par le médecin et/ou par le patient lui-même. Cette évaluation peut être réalisée par toute méthode, de manière commode par examen visuel ou au toucher, en utilisant par exemple la grille de lecture consensus de l'EAACI (voir Allergy 61 (2006) 1377-1384). Ainsi, il existe différents degrés dans la réaction inflammatoire cutanée : érythème - érythème et infiltrations - érythèmes et quelques papules - érythème pourvu de nombreuses papules - érythème à vésicules. Une réaction inflammatoire appropriée est typiquement une réaction inflammatoire de type III, c'est-à-dire comprenant un érythème et quelques papules. Dans la mesure du possible, c'est ce type de réaction que l'on essaie d'entretenir au cours du traitement. Bien entendu, lorsque le patient développe une réaction moins forte dès le départ, la réaction de référence sera nécessairement moins forte, et elle sera adaptée patient par patient. Par ailleurs, au cours du traitement, la réaction inflammatoire diminuera en raison de la désensibilisation du sujet.
Pour la mise en oeuvre de l'invention, il est également possible d'évaluer la réaction inflammatoire par dosage de marqueurs de l'inflammation, comme des cytokines ou interférons.
Afin d'ajuster le mieux possible le traitement, dans un mode de réalisation particulier de l'invention, la réactivité inflammatoire est évaluée une ou plusieurs fois au cours du traitement par mise en contact du sujet avec une gamme de doses de l'allergène, permettant de sélectionner la dose la mieux adaptée pour la suite du traitement. L'évaluation de la réaction inflammatoire peut être réalisée tout au long du traitement, c'est-à-dire à chaque nouvelle application de l'allergène.
Il est également possible de ne réaliser cette évaluation que à certains intervalles, et non pas à chaque application.

En outre, s'il est préféré d'entretenir une réaction inflammatoire tout au long du traitement, cela n'est pas obligatoire et une désensibilisation efficace peut également être obtenue lorsque le contact immunitaire a été établi une première fois et que les réactions en cascades spécifiques (activation des cellules immunologiquement compétentes et production de cytokines et d'anticorps) ont été suffisamment enclenchées. Ainsi, dans la pratique, lorsque la réaction inflammatoire a atteint un niveau suffisant se traduisant par exemple par une réaction cutanée de l'intensité voulue, il est possible de retirer le dispositif, puis d'en remettre un autre après un délai à définir, dans une autre zone cutanée. Dans une variante de l'invention, l'allergène (ou le dispositif d'application) est maintenu sur la peau au moins jusqu'à ce que la réaction inflammatoire atteigne un degré de référence. Lorsque ce degré est atteint, l'allergène (ou le dispositif) peut être retiré, et une période de latence est observée avant une nouvelle application. La réaction de référence est typiquement une réaction visible à l'oeil nu, par exemple sous forme de lésions érythémateuses infiltrées ou prenant un aspect eczématiforme.

L'allergène (ou le dispositif) peut également être maintenu sur la peau de manière à entretenir la réaction inflammatoire. Typiquement, dans ce cas, le dispositif d'application est retiré lorsque tout l'allergène aura diffusé, ou si la réaction inflammatoire atteint une trop forte intensité.

Il est entendu, que, au cours du traitement, ces variantes peuvent être alternées.

Dans un mode de mise en oeuvre, on laisse s'écouler une période de temps entre la pose de dispositifs comprise entre 1 et 21 jours.

Le traitement peut être considéré comme terminé et mené avec succès lorsque toute réactivité cutanée à l'allergène aura disparu ou aura été très significativement réduite.

Le succès de la cure pourra être confirmé par un test de provocation orale, ou par tout autre moyen reconnu en allergologie. En conséquence, la durée de la cure est variable et dépend de l'évolution de la réactivité cutanée à l'allergène. L'objectif de la cure de désensibilisation selon la méthode de l'invention est soit de faire disparaître entièrement la réaction cutanée locale à l'allergène après contact prolongé, soit de minorer la réactivité de telle sorte que l'on estime que la réactivité du sujet à l'allergène ayant été significativement réduite par la cure d'immunothérapie épicutanée, le risque de choc anaphylactique lors d'un contact accidentel avec l'allergène se trouve significativement réduit.

L'invention peut être mise en oeuvre avec tout dispositif d'administration cutanée assurant le contact de la peau avec l'allergène. Il s'agit de préférence de dispositifs de type patch, timbres ou pansements, de préférence de patchs, par exemple de patchs occlusifs.

Un tel dispositif comprend typiquement un support sur lequel est fixé l'allergène, et/ou un réservoir ou une chambre comprenant l'allergène.

Le dispositif utilisé est typiquement de nature transdermique à diffusion passive, c'est-à-dire qu'il ne contient pas de moyens pour causer une perforation ou une abrasion de la peau. En effet, les données obtenues montrent que le procédé de l'invention permet d'induire une désensibilisation épicutanée même sans traitement préalable de la peau. Toutefois, il est également possible d'utiliser un dispositif pourvu de propriétés visant à augmenter la perméabilité des couches superficielles de la peau à l'allergène et/ou à augmenter la réactivité cutanée à l'aide de moyen physique, chimique ou biologique. Ainsi, bien que cela ne soit pas essentiel, il est possible de pré-traiter la peau (par exemple par traitement abrasif) avant chaque application du dispositif, pour favoriser encore le traitement de désensibilisation.

Dans un mode de mise en oeuvre préféré, on utilise un dispositif de type patch occlusif, comme par exemple un patch électrostatique Viaskin^{®} décrit dans la demande de brevet WO02/071950.

Par ailleurs, dans un mode de mise en oeuvre préféré, le dispositif est pourvu de moyens assurant son maintien sur la peau. Tout moyen adhésif peut être utilisé à cet effet, y compris des matériaux de type gomme, polymère ou plastique ayant un fort pouvoir adhésif. Il est également possible d'utiliser un dispositif muni d'un bracelet, comme décrit par exemple dans la demande n° FR0753787, ou d'une feuille d'exfoliation, comme décrit par exemple dans la demande n° FR0753265.

Il est entendu que la nature et la forme précises du dispositif peuvent être adaptées par l'homme du métier, la propriété requise étant d'assurer le contact entre l'allergène et la peau au niveau du site d'application.

Dans un mode particulier de mise en oeuvre, le traitement est réalisé au moyen d'un dispositif muni d'un fond (« backing ») au moins partiellement transparent (par exemple un dispositif VIASKIN^{®}), de sorte que la réaction inflammatoire (par exemple l'apparition d'un érythème) peut être observée directement à travers la membrane transparente. Le backing du dispositif peut même être adapté pour aider le patient à déterminer le niveau de seuil de réaction cutanée à partir duquel il pourra retirer le dispositif. Ainsi, dans un mode de réalisation particulièrement avantageux, on utilise un dispositif dont le fond présente une zone transparente sur laquelle est imprimée un motif de suivi, par exemple une grille rouge ou des cercles concentriques rouges. Lorsque la peau n'a pas réagi, les dessins sont bien visibles et ils s'effacent lorsque la réaction cutanée est obtenue. Le recours à un système de backing transparent présente de nombreux avantages, et notamment :
- cela évite les contacts inutilement prolongés avec l'allergène, améliore la tolérance cutanée, minimise les effets secondaires désagréables de type prurit et par le fait améliorent l'acceptabilité et l'observance ;
- cela augmente les performances de la méthode en terme de sécurité et minimise encore les risques de réaction systémique qui ont été fatales à nombre de méthodes de désensibilisation à l'arachide par le passé ;
- cela offre une solution qui permet d'intégrer la notion de temps variable d'exposition à l'allergène dans le procédé industriel.

Dans ce contexte, un objet de l'invention concerne un dispositif cutanée pour l'application d'un allergène sur la peau d'un sujet, caractérisé en ce qu'il comprend un fond muni d'une zone transparente permettant de visualiser la réaction cutanée inflammatoire provoquée par l'application de l'allergène sur la peau. Avantageusement, le dispositif est un dispositif de type occlusif et/ou à diffusion passive. Le dispositif est typiquement un patch.

Un autre objet de l'invention concerne par ailleurs un kit comprenant plusieurs dispositifs d'application cutanée d'un allergène, les dispositifs comprenant différentes doses de l'allergène et/ou différentes formes de l'allergène.
L'allergène utilisé dans le cadre de l'invention peut être de nature, forme et origine variées. Ainsi, il peut s'agir d'un allergène alimentaire, respiratoire ou cutanée, par exemple. Il peut s'agir de protéines (ou de polypeptides ou peptides), lipides, sucres, etc., qui peuvent être sous forme de produits d'extraction, sous forme recombinante et/ou d'origine synthétique, par exemple, le cas échéant modifiés chimiquement ou immunologiquement pour modifier leurs propriétés. Les allergènes peuvent êtres sous forme entière, native, ou sous forme fragmentée, dénaturée, etc. On utilise typiquement une préparation comprenant plusieurs antigènes allergéniques en combinaison, et/ou un ou des allergènes sous forme de conjugués ou de complexes. Il est entendu que la méthode de l'invention est essentiellement indépendante de la nature de l'allergène, et est donc applicable à toute préparation comprenant des allergènes, comme par exemples des allergènes alimentaires (e.g., arachide, crustacés, lait, oeuf, poissons, blé, soja, et.) ou des allergènes cutanés ou respiratoires (acariens, pollen, poils d'animaux, etc.).

L'allergène est, avantageusement, formulé avec tout excipient ou véhicule adapté à un usage pharmaceutique. La préparation allergénique mise en oeuvre peut se présenter sous différentes formes, comme par exemple liquide ou solide. Dans un mode préféré, l'allergène est sous forme sèche, c'est-à-dire sous une forme déshydratée, et notamment une forme de poudre, particules, agglomérats, galette, forme séchée, etc. La forme sèche peut être obtenue par lyophilisation, ou par simple évaporation.

Dans un mode de mise en oeuvre particulier, l'allergène est sous forme de poudre.

Comme indiqué précédemment, un avantage de l'invention est qu'elle permet d'obtenir une désensibilisation à un allergène même sans co-administration d'adjuvant. Aussi, dans un mode de mise en oeuvre préféré, la préparation allergénique appliquée sur la peau du patient est dépourvue de tout composé adjuvant ajouté. Le terme adjuvant désigne au sens de l'invention tout composé qui exerce une action physico-chimique et/ou biologique sur la peau, facilitant la pénétration de l'antigène dans la peau, et qui augmente la réponse immunitaire contre l'antigène. Par action physico-chimique et/ou biologique, l'adjuvant tel que défini ici est un composé qui altère la perméabilité de l'épiderme par perturbation de la couche cornée, permettant ainsi le passage de l'antigène et son contact avec les cellules immunologiquement actives de l'épiderme. Des exemples de composés adjuvants sont notamment la toxine cholérique (CT) ou la LT (toxine thermolabile d'E-coli). D'autres exemples sont les transfersomes, décrits par Paul et al (Paul A, Cevc G, Bachhawat BK. Transdermal immunization with large proteins by ultradeformable drug carriers. Eur J Immunol. 1995, 25 (12) :3521-4), qui permettent de transporter un antigène dans les membranes.

Dans un mode normal de mise en oeuvre, le dispositif contient une quantité d'allergène comprise entre 0,1 et 1000 µg/cm2 de surface du dispositif, de préférence entre 20 et 500 µg par cm2, plus préférentiellement entre 20 et 200 µg par cm2. Il est entendu que cette quantité peut être adaptée par l'homme du métier en fonction des objectifs, de la pathologie, de la durée du traitement, du sujet, etc.

Les exemples expérimentaux qui suivent montrent qu'une telle diminution de la réactivité peut être atteinte en utilisant un contrôle des doses selon l'invention. La méthode d'immunothérapie épicutanée de l'invention comporte de nombreux avantages par rapport à l'état actuel de l'art.

Il s'agit d'une méthode originale visant à provoquer et contrôler une réaction inflammatoire en appliquant régulièrement et durablement un allergène sur la peau d'un sujet allergique audit allergène dans le but de diriger la réaction de l'organisme vis-à-vis de cet allergène vers la tolérance.
Il s'agit d'une méthode parfaitement sûre comme le confirme l'expérience accumulée dans le domaine des atopy patch tests ainsi que les données recueillies depuis l'introduction du DIALLERTEST^{®} en France, premier atopy patch test prêt à l'emploi diffusé à plus de 100000 exemplaires. De part cette grande sécurité d'emploi, la méthode selon l'invention permet de désensibiliser des sujets allergiques à des allergènes tels que l'arachide ou les crustacés qui ne sont pas pris en charge par les méthodes classiques de désensibilisation en raison des risques encourus.
L'immunothérapie peut être interrompue à tout moment en retirant le dispositif appliqué sur la peau, ce qui n'est pas envisageable avec les méthodes traditionnelles de désensibilisation au cours desquelles l'antigène est administré dans l'organisme.
La première étape de la méthode permet d'une part de confirmer l'éligibilité du sujet à l'immunothérapie épicutanée (les sujets présentant une réaction cutanée sont éligibles) et d'autre part de mesurer le degré de réactivité cutanée du sujet à l'allergène afin de guider au mieux la conduite de la cure d'immunothérapie épicutanée. Il est ainsi possible de choisir le protocole le plus adapté au patient afin d'optimiser la tolérance cutanée et de maintenir le meilleur niveau d'efficacité.
Au cours de la cure, il est possible d'ajuster la posologie et/ou la fréquence d'administration en fonction de l'évolution de la réactivité cutanée, mesurée en continu. Il est ainsi possible d'optimiser en permanence l'action thérapeutique et de maintenir le plus haut niveau d'efficacité tout au long du traitement.
La durée de la cure dépend des objectifs thérapeutiques fixés au début de la cure et de l'évolution de la réactivité cutanée à l'allergène. La cure est considérée comme terminée lorsque par exemple sont atteints les objectifs de réduction de réactivité à l'allergène constatés sur la peau et confirmés par un test de provocation ou tout autre moyen de l'allergologie ou lorsque l'extinction complète de réactivité cutanée est constatée.
D'autres aspects et avantages de l'invention apparaîtront à la lecture des exemples qui suivent, qui doivent être considérés comme illustratifs et non limitatifs.

### Légende des Figures :

Figure 1 : Résultats de pléthysmographie obtenus après 8 semaines (a) ou 16 semaines (b) de désensibilisation au pollen de Dactyle. C : contrôle, ND : non désensibilisé, SCD : désensibilisation sous-cutanée, EPD : désensibilisation épicutanée.
Figure 2 : Résultats de pléthysmographie obtenus après 8 semaines (a) ou 16 semaines (b) de désensibilisation à l'ovalbumine. C : contrôle, ND : non désensibilisé, SCD : désensibilisation sous-cutanée, EPD : désensibilisation épicutanée.
Figure 3 : Suivi du taux d'anticorps avant et après 8 semaines de désensibilisation avec des protéines d'arachide. Graphe de gauche : IgE spécifiques, graphe de droite : IgG1 et IgG2a spécifiques. EP : épicutanée, ID : intradermique, ND : non désensibilisé.
Figure 4 : Histamine libérée après test de provocation. EP : épicutanée, ID : intradermique, ND : non désensibilisé. Les résultats sont exprimés en nM.
Figure 5 : Evolution du taux des IgE spécifiques (V0, prélèvement initial, V1, prélèvements à un mois, V3, prélèvement à 3 mois).
Figure 6 : Visualisation de la réaction inflammatoire cutanée au cours du traitement : A : marquée ; B : moyenne ; C : atténuée.
Figure 7A : Activation des cellules de Langerhans et afflux des cellules inflammatoires après 24h et 48h d'application d'un allergène à l'aide de VIASKIN^{®}. Après 24h d'application, les cellules de Langerhans sont activées et commencent leur migration vers les ganglions lymphatiques. Après 48h, les cellules inflammatoires sont alertées et des granulomes inflammatoires sont bien visibles.
Figure 7B : Evolution de la réponse cellulaire locale après différents temps d'application d'un allergène (ovalbumine) à l'aide de VIASKIN^{®}. Après 6 heures d'application, la réponse cellulaire n'est pas perceptible. Après 24h, l'élévation du taux local des cellules inflammatoires se poursuit après le retrait du VIASKIN^{®}. Cette évolution est plus forte après 48h d'application.
Figure 8 : Evolution du profil de réponse locale après application prolongée (6 et 12 semaines) d'un allergène sur la peau d'un animal sensibilisé à l'aide du VIASKIN^{®}. Après application répétée de 24h, la réponse est de type TH2 prédominant. Après application répétée de 48h, la réponse est mixte, puis, TH1 prédominante.

### Exemples

### Exemple 1. Désensibilisation à l'ovalbumine et au pollen

### 1.1. Méthodologie

Dans le cas de l'OVA et de POL, des souris BALB/c ont été sensibilisées 2 semaines de suite (J0 et J7) par une injection sous-cutanée de 10 µg d'allergène associé à de l'hydroxyde d'aluminium. A J14, les souris ont reçu une instillation nasale de 10 µg d'allergène seul.

A partir de J21, les souris ont suivi ou non un traitement de désensibilisation. Les souris traitées ont reçu 1 fois par semaine pendant 8 semaines 100 µg d'allergène administré soit par voie épicutanée, grâce au système VIASKIN^{®}, dans des conditions assurant l'entretien de la réaction inflammatoire cutanée, soit par voie sous-cutanée en association à l'hydroxyde d'aluminium (contrôle positif). Les souris non désensibilisées ont été maintenues dans les conditions normales d'animalerie.

L'évolution du taux d'anticorps (IgE et IgG spécifiques) a été suivie sur les sérums des souris prélevés après 8 semaines et 16 semaines de désensibilisation.

L'efficacité du traitement de désensibilisation OVA et POL a été également étudiée grâce à des mesures de pléthysmographie permettant l'évaluation de l'hyperréactivité bronchique des souris désensibilisées ou non.

### 1.2. Résultats

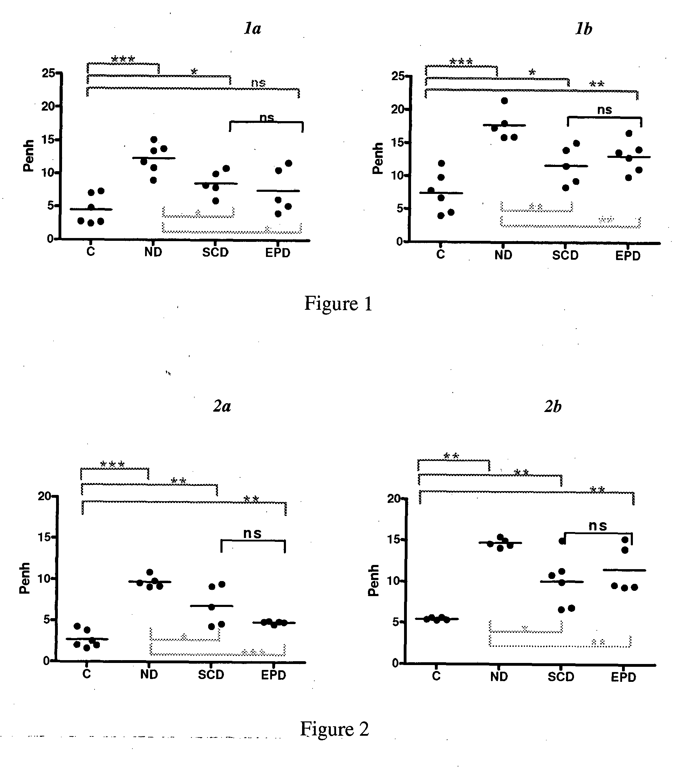

L'hyperréactivité bronchique a été mesurée pour les souris traitées ou non à l'aide du VIASKIN^{®} (figures 1 et 2).

L'hyperréactivité bronchique des souris traitées par le VIASKIN^{®} dans des conditions assurant l'entretien de la réaction inflammatoire cutanée, est significativement réduite, de 3,7 à 2,6 fois par rapport aux souris non traitées. L'hyperréactivité bronchique des souris traitées par voie sous-cutanée n'est pas significativement différente de celle des souris traitées par le VIASKIN^{®}.

### Exemple 2. Désensibilisation à l'arachide

### 2.1. Méthodologie

Pour l'ARA, les souris ont été sensibilisées sur 6 semaines par une administration intra-gastrique tous les 6 jours (J1, J7, J13, J19, J25, J33) de 1 mg de protéines d'ARA associées à 10 µg de toxine cholérique.

Le protocole de désensibilisation débute à J75 pour une période de 8 semaines. Les souris sont désensibilisées une fois par semaine en administrant 100 µg de formulation ARA par voie intradermique ou par voie épicutanée grâce au système VIASKIN^{®}, dans des conditions assurant le maintien de la réaction inflammatoire. Des souris non désensibilisées ne suivent aucun traitement particulier après sensibilisation.

L'efficacité de la désensibilisation est suivie par la mesure du taux d'anticorps (IgE, IgG1 et IgG2a spécifiques) ainsi que par la mesure de la libération d'histamine après des tests de provocation oraux. L'histamine est un médiateur libéré lors de la dégranulation des cellules immunitaires type mastocytes ou basophiles. Cette dégranulation est un marqueur de l'induction d'une réponse allergique.

### 2.2. Résultats

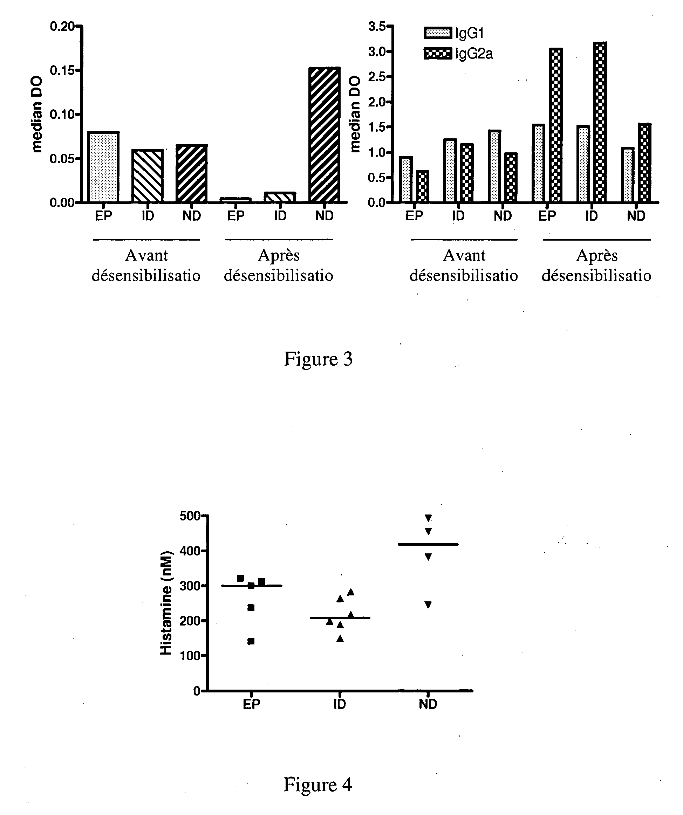

L'analyse du traitement de désensibilisation ARA a été validée par une analyse des taux anticorps spécifiques chez les souris (figure 3). Les souris désensibilisées par voie épicutanée ou par voie intradermique présentent une diminution du taux d'IgE spécifiques (marqueur de l'allergie à l'ARA) et une augmentation du taux d'IgG2a spécifiques. Les taux d'IgE spécifiques détectables chez les souris non désensibilisées restent élevés et les taux d'IgG2a spécifiques sont plus faibles que chez les souris désensibilisées. Aucune différence significative n'est observée entre les voies de désensibilisation épicutanée et intradermique.

Des tests de provocation oraux ont été réalisés sur les souris désensibilisées ou non. Les souris désensibilisées par voie intradermique ou par voie épicutanée présentent une plus faible libération d'histamine que les souris non désensibilisées. Ce résultat montre une diminution de la réactivité allergique des souris désensibilisées.

### Discussion

A travers les différents modèles d'étude et les différents allergènes étudiés, il apparaît que :
- La méthode d'immunothérapie épicutanée basée sur l'entretien de la réponse inflammatoire permet de déclencher une réponse clinique et biologique de type systémique ;
- Les animaux ayant bénéficié de la méthode d'immunothérapie épicutanée basée sur l'entretien de la réponse inflammatoire après sensibilisation préalable par voie orale ou cutanée ont une réponse atténuée aux tests de provocation, comparable à celle des animaux ayant subi une immunothérapie par voie sous cutanée ou intradermique ;
   La réponse sérologique obtenue confirme l'induction d'un processus de tolérance par l'immunothérapie épicutanée.

### Exemple 3. Données Cliniques

Caractéristique du patient : Enfant, 6 ans 5/12, 18.3kg. Diarrhée chronique, vomissements, eczéma généralisée. Prick test positif au lait de vache. IgE totales 486UI. IgE spécifiques lait de vache 32.7UI.

Test de provocation au lait positif à 0.6 ml (prurit laryngé, toux, douleurs abdominales, urticaire).

Traitement : Application de VIASKIN^{®} contenant 130µg de protéines de lait, renouvelée tous les deux jours pendant 3 mois dans des conditions assurant la persistance d'une réaction cutanée au contact de l'allergène.

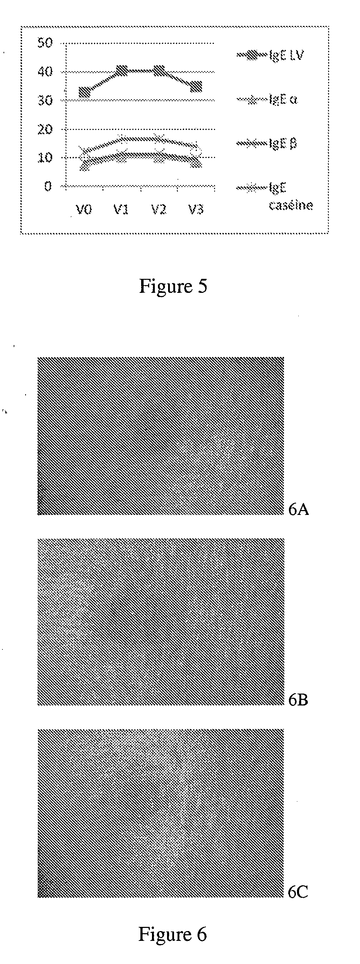

Résultats : Les résultats sont présentés dans les figures 5 et 6. Ils illustrent une amélioration très substantielle du seuil de tolérance au lait de vache, corrélée au contrôle de la réaction inflammatoire au cours du traitement. En fin de cure d'immunothérapie, une atténuation de la réaction cutanée est constatée. Le test de provocation orale à 3 mois montre un seuil de tolérance jusqu'à 64.6 ml sans aucune réaction clinique.

### Exemple 4. Evolution de la réponse cellulaire suite à l'application d'un allergène chez la souris sensibilisée

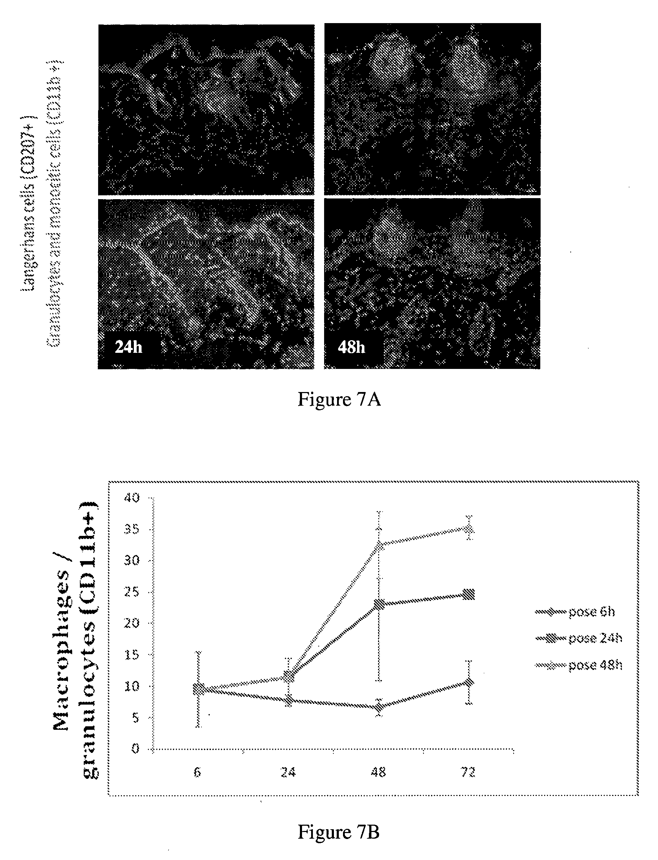

L'application d'un allergène sur la peau de la souris préalablement sensibilisée, active les cellules de Langerhans et leur migration vers les ganglions lymphatiques (Figure 7A).
Dans le même temps, les cellules activées produisent des cytokines pro-inflammatoires qui attirent les cellules de l'inflammation vers les couches superficielles de la peau. Cette réaction « de première phase » se produit chez le sujet sensibilisé car il possède des cellules à mémoire et des IgE spécifiques dirigés contre l'allergène. Au contact de l'allergène, il se produit donc une réaction initiale de rejet de l'allergène.

Dans un deuxième temps, une réaction inflammatoire locale caractérisée par l'afflux des cellules de l'inflammation aigue (mastocytes, macrophages) et chroniques (leucocytes) apparaît (Figures 7A et 7B). Chez la souris préalablement sensibilisée, l'application d'un allergène (ovalbumine) sur la peau avec Viaskin^{®} se traduit par une réaction faiblement inflammatoire après 24h de contact et par contre massivement inflammatoire après 48h de contact avec envahissement de la zone cutanée par des polynucléaires. C'est cette réaction, plus complexe, qui est ciblée par le traitement. Dans une étude menée chez la souris sensibilisée, l'application pendant 6h, 24h et 48h de l'allergène détermine une réaction inflammatoire (matérialisée ici par l'élévation locale du taux des macrophages) plus importante après 48h d'application (Figure 7B).

### Discussion :

Au cours du traitement de désensibilisation épicutanée, il est visé de reproduire une réaction inflammatoire cellulaire dont le rôle est indispensable dans le processus de tolérance.
En réalité, ce n'est pas l'intensité de la réaction inflammatoire qui varie avec le temps mais surtout sa nature elle-même.
Des études en immunofluorescence confirment le rôle central de l'inflammation et l'aspect complètement différent du profil de la réaction inflammatoire en fonction de la durée d'application.
La durée d'application de l'allergène est un facteur essentiel pour provoquer une réaction cellulaire.

### Exemple 5. Evolution du profil de réponse locale après application prolongée d'un allergène

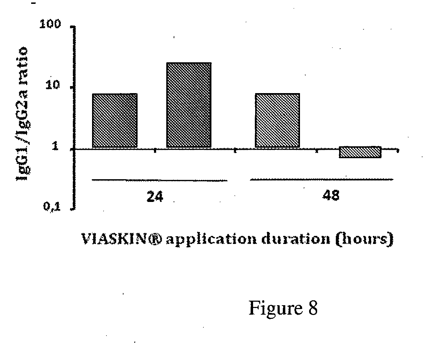

La durée d'application n'est pas une condition suffisante pour obtenir le type de réaction thérapeutique (tolérogène) recherchée. Chez la souris, l'administration répétée d'un allergène sur la peau entraine une élévation des anticorps spécifiques de cet allergène que l'application soit « courte » (24h) ou « longue » (48h). Lorsque l'application est poursuivie (jusqu'à 88 jours), le taux des anticorps est toujours aussi élevé mais la proportion relative des lymphocytes Th1/Th2 est modifiée dans le sens d'une élévation des Th1 chez les sujets recevant des Viaskin^{®} pendant 48h (Figure 8).

### Discussion

Les données expérimentales mettent en évidence que la méthode selon l'invention vise à induire une réaction dont les caractéristiques sont différentes de la réaction habituellement obtenue par le simple dépôt de l'allergène sur la peau chez l'allergique. Seule la réaction obtenue après application « longue » au moyen de Viaskin^{®} permet d'obtenir un effet « tolérogène » matérialisé par une activation des cellules lymphocytaires Th1 alors que la réaction obtenue après une application « courte » est de type Th2.
Au cours de la cure de désensibilisation par Viaskin^{®}, l'application répétée de l'allergène sur la peau intacte chez le patient préalablement sensibilisé, provoque une réaction inflammatoire cellulaire telle qu'elle est couramment observée en clinique avec les atopy patch tests.
En provoquant et en entretenant la réaction inflammatoire par l'application répétée et prolongée de l'allergène, on crée les conditions d'inflammation locales spécifiques. Au cours de cette réaction, l'équilibre est déplacé en faveur des lymphocytes Th1.

Il apparait donc que l'application prolongée et répétée d'un allergène sur la peau est susceptible non seulement d'entraîner une réaction inflammatoire mais également d'orienter cette réaction.

## Revendications

1. Utilisation d'un allergène pour la préparation d'une composition pour la désensibilisation épicutanée d'un sujet allergique au dit allergène, comprenant l'application répétée de l'allergène sans adjuvant sur la peau du sujet au moyen d'un dispositif cutané non perforant assurant un contact entre l'allergène et la peau dans des conditions de dose, ou de fréquence ou de temps d'application, ou de forme de l'allergène permettant de provoquer une réaction inflammatoire cutanée sur la zone d'application de l'allergène et d'entretenir ladite réaction inflammatoire au cours du traitement.

2. Utilisation selon la revendication 1, pour modifier dans le sens de la tolérisation la réponse immunitaire d'un sujet allergique au dit allergène.

3. Utilisation selon l'une quelconque des revendications précédentes, **caractérisée en ce que** le degré de la réaction inflammatoire est évalué par examen visuel.

4. Utilisation selon l'une quelconque des revendications précédentes, **caractérisée en ce que**, lorsque la réaction inflammatoire diminue au cours du traitement, la dose et/ou la fréquence et/ou le temps d'application de l'allergène sont augmentés ou ; lorsque la réaction inflammatoire augmente au cours du traitement, la dose et/ou la fréquence et/ou le temps d'application de l'allergène sont diminués.

5. Utilisation selon l'une quelconque des revendications précédentes, **caractérisée en ce que** l'allergène est appliqué sur la peau intacte du sujet.

6. Utilisation selon l'une quelconque des revendications précédentes, **caractérisée en ce que** l'allergène est sous forme sèche.

7. Utilisation selon l'une quelconque des revendications précédentes, **caractérisée en ce que** ledit dispositif cutanée assurant un contact entre l'allergène et la peau est un dispositif à diffusion passive, de préférence un dispositif occlusif cutanée.

8. Utilisation selon l'une quelconque des revendications précédentes, **caractérisée en ce que** l'allergène ou le dispositif est maintenu sur la peau au moins jusqu'à ce que la réaction inflammatoire atteigne un degré de référence.

9. Utilisation selon la revendication 8, **caractérisée en ce que** le degré de référence est une réaction inflammatoire visible à l'oeil nu, par exemple sous forme de lésions érythémateuses infiltrées ou prenant un aspect eczématiforme.

10. Utilisation selon l'une quelconque des revendications précédentes, **caractérisée en ce que** le fond du dispositif comprend une zone transparente permettant de visualiser la réaction inflammatoire, de préférence sur laquelle une grille est imprimée permettant d'évaluer le degré de la réaction inflammatoire.

11. Utilisation selon l'une quelconque des revendications précédentes, **caractérisée en ce qu'**elle comprend une étape préalable de détermination de la réaction inflammatoire initiale du sujet allergique à une concentration prédéterminée de l'allergène.

12. Utilisation selon l'une quelconque des revendications précédentes, **caractérisée en ce qu'**elle comprend, en début et/ou en cours de traitement, une étape de détermination de la réaction inflammatoire du sujet à différentes doses de l'allergène ou à différentes formes de l'allergène, pour sélectionner la dose ou la forme thérapeutique.

13. Utilisation selon l'une quelconque des revendications précédentes, **caractérisée en ce que** l'allergène est un allergène alimentaire, respiratoire ou cutanée.

14. Utilisation de la réaction cutanée inflammatoire générée par l'application d'un allergène sur la peau d'un patient, comme marqueur pour adapter le protocole de désensibilisation épicutanée dudit patient au dit allergène.

15. Allergène pour son utilisation pour la désensibilisation épicutanée d'un sujet allergique au dit allergène par application répétée de l'allergène sur la peau du sujet au moyen d'un dispositif cutané occlusif non perforant assurant un contact entre l'allergène et la peau, l'allergène étant utilisé sans composé adjuvant et dans des conditions de dose, ou de fréquence ou de temps d'application, ou de forme de l'allergène suffisantes pour provoquer une réaction inflammatoire cutanée sur la zone d'application de l'allergène et entretenir ladite réaction inflammatoire au cours du traitement.

16. Allergène pour son utilisation selon la revendication 15, **caractérisé en ce qu'**il est sous forme sèche.

17. Allergène pour son utilisation selon la revendication 15 ou 16, **caractérisé en ce que** l'allergène est appliqué sur la peau intacte du sujet.

18. Allergène pour son utilisation selon la revendication 15 ou 16, **caractérisé en ce que** l'allergène est appliqué après traitement abrasif de la peau.

## Patentansprüche

1. Verwendung eines Allergens zur Herstellung einer Zusammensetzung zur epikutanen Desensibilisierung einer Person, die gegen das Allergen allergisch ist, umfassend die wiederholte Anwendung des Allergens ohne Adjuvans auf die Haut der Person mittels einer nicht durchlässigen Hautvorrichtung, welche den Kontakt zwischen dem Allergen und der Haut gewährleistet, unter Bedingungen der Dosis, der Häufigkeit oder der Anwendungszeit oder der Form des Allergens, die das Hervorrufen einer entzündlichen Hautreaktion im Anwendungsbereich des Allergens und das Aufrechterhalten der entzündlichen Reaktion im Laufe der Behandlung erlaubten.

2. Verwendung nach Anspruch 1 zur Veränderung der Immunantwort einer Person, die gegen das Allergen allergisch ist, im Sinne der Tolerierung.

3. Verwendung nach einem der vorherigen Ansprüche, **dadurch gekennzeichnet, dass** der Grad der entzündlichen Reaktion durch visuelle Untersuchung bewertet wird.

4. Verwendung nach einem der vorherigen Ansprüche, **dadurch gekennzeichnet, dass** die Dosis und/oder Häufigkeit und/oder die Zeit der Anwendung erhöht werden, wenn die entzündliche Reaktion im Laufe der Behandlung nachlässt oder, wenn die entzündliche Reaktion im Laufe der Behandlung zunimmt, die Dosis und/oder die Häufigkeit und/oder die Zeit der Anwendung des Allergens verringert werden.

5. Verwendung nach einem der vorherigen Ansprüche, **dadurch gekennzeichnet, dass** das Allergen auf die intakte Haut der Person angewendet wird.

6. Verwendung nach einem der vorherigen Ansprüche, **dadurch gekennzeichnet, dass** das Allergen in trockener Form vorliegt.

7. Verwendung nach einem der vorherigen Ansprüche, **dadurch gekennzeichnet, dass** die Hautvorrichtung, welche den Kontakt zwischen dem Allergen und der Haut gewährleistet, eine Vorrichtung mit passiver Diffusion, vorzugsweise eine Hautverschlussvorrichtung ist.

8. Verwendung nach einem der vorherigen Ansprüche, **dadurch gekennzeichnet, dass** das Allergen oder die Vorrichtung wenigstens so lange auf der Haut bleibt, bis die entzündliche Reaktion einen Referenzgrad erreicht.

9. Verwendung nach Anspruch 8, **dadurch gekennzeichnet, dass** der Referenzgrad eine entzündliche Reaktion ist, die mit nacktem Auge sichtbar ist, beispielsweise in Form von eingedrungenen erythematösen Läsionen oder unter Annehmen eines ekzematoiden Aussehens.

10. Verwendung nach einem der vorherigen Ansprüche, **dadurch gekennzeichnet, dass** der Boden der Vorrichtung eine durchsichtige Zone umfasst, die es erlaubt, die entzündliche Reaktion zu visualisieren, vorzugsweise ist darauf ein Raster gedruckt, das es gestattet, den Grad der entzündlichen Reaktion zu bewerten.

11. Verwendung nach einem der vorherigen Ansprüche, **dadurch gekennzeichnet, dass** sie einen vorhergehenden Schritt der Bestimmung der entzündlichen Erstreaktion der allergischen Person auf eine vorbestimmte Konzentration des Allergens umfasst.

12. Verwendung nach einem der vorherigen Ansprüche, **dadurch gekennzeichnet, dass** sie, zu Beginn oder im Laufe der Behandlung einen Schritt der Bestimmung der entzündlichen Reaktion der Person auf unterschiedliche Dosen des Allergens oder auf unterschiedliche Formen des Allergens umfasst, um die therapeutische Dosis oder Form zu wählen.

13. Verwendung nach einem der vorherigen Ansprüche, **dadurch gekennzeichnet, dass** das Allergen ein Lebensmittel-, ein Atmungs- oder ein Hautallergen ist.

14. Verwendung der entzündlichen Hautreaktion, die bei der Anwendung eines Allergens auf die Haut eines Patienten erzeugt wird, als Marker zur Anpassung des epikutanen Desensibilierungsprotokolls des Patienten an das Allergen.

15. Allergen zur Verwendung zur epikutanen Desensibilisierung einer auf das Allergen allergischen Person durch wiederholte Anwendung des Allergens auf die Haut der Person mittels einer undurchlässigen Hautverschlussvorrichtung, die einen Kontakt zwischen dem Allergen und der Haut gewährleistet, wobei das Allergen ohne Adjuvansverbindung verwendet wird und unter Bedingungen der Dosis oder der Häufigkeit oder der Zeit der Anwendung oder der Form des Allergens, die ausreichen, um eine entzündliche Hautreaktion im Anwendungsbereich des Allergens hervorzurufen und die entzündliche Reaktion im Laufe der Behandlung beizubehalten.

16. Allergen zur Verwendung nach Anspruch 15, **dadurch gekennzeichnet, dass** das Allergen in trockener Form vorliegt.

17. Allergen zur Verwendung nach Anspruch 15 oder 16, **dadurch gekennzeichnet, dass** das Allergen auf die intakte Haut der Person angewendet wird.

18. Allergen zur Verwendung nach Anspruch 15 oder 16, **dadurch gekennzeichnet, dass** das Allergen nach einer abschleifenden Behandlung der Haut angewendet wird.

## Claims

1. The use of an allergen for the preparation of a composition for the epicutaneous desensitization of a subject allergic to said allergen, comprising the repeated application of the allergen without an adjuvant on the skin of the subject using a non-perforating cutaneous device ensuring a contact between the allergen and the skin under conditions of dose, frequency or duration of application, or of form of the allergen, allowing to provoke of a cutaneous inflammatory reaction on the application zone of the allergen and to sustain said inflammatory reaction during the course of the treatment.

2. The use of claim 1, to modify in the sense of tolerisation the immune response of a subject allergic to said allergen.

3. The use according to anyone of the preceding claims, wherein the degree of the inflammatory reaction is evaluated by visual examination.

4. The use according to anyone of the preceding claims, wherein, when the inflammatory reaction decreases during the course of the treatment, the dose and/or the frequency and/or the duration of application of the allergen are increased or; when the inflammatory reaction increases during the course of the treatment, the dose and/or the frequency and/or the duration of application of the allergen are decreased.

5. The use according to anyone of the preceding claims, wherein the allergen is applied on an intact skin of the subject.

6. The use according to anyone of the preceding claims, wherein the allergen is in dry form.

7. The use according to anyone of the preceding claims, wherein the cutaneous device ensuring a contact between the allergen and the skin is a passive diffusion device, preferably a cutaneous occlusive device.

8. The use according to anyone of the preceding claims, wherein the allergen or device is maintained on the skin at least until the inflammatory reaction reaches a reference level.

9. The use according to claim 8, wherein the reference level is an inflammatory reaction visible at naked eye, for instance in the form of infiltrated erythematous lesions or having an eczematiform aspect.

10. The use according to anyone of the preceding claims, wherein the backing of the device comprises a transparent zone allowing to visualize the inflammatory reaction, preferably onto which a grid is printed allowing to evaluate the degree of the inflammatory reaction.

11. The use according to anyone of the preceding claims, which comprises a prior step of determining the initial inflammatory reaction of the allergic subject to a predetermined concentration of the allergen.

12. The use according to anyone of the preceding claims, comprising, at the beginning and/or during the course of the treatment, a step of determining the inflammatory reaction of the subject to different doses of the allergen or to different forms of the allergen, in order to select the therapeutic dose or form.

13. The use according to anyone of the preceding claims, wherein the allergen if a food, respiratory or cutaneous allergen.

14. The use of the cutaneous inflammatory reaction generated by the application of an allergen on the skin of a patient, as a marker to adapt the protocol of epicutaneous desensitization to said allergen of said patient.

15. An allergen for use for the epicutaneous desensitization of a subject allergic to said allergen by repeated application of the allergen on the skin of the subject using a non-perforating occlusive cutaneous device ensuring a contact between the allergen and the skin, the allergen being used without an adjuvant compound and under conditions of dose, or of frequency or of duration of application, or of form of the allergen, sufficient to provoke of a cutaneous inflammatory reaction on the application zone of the allergen and to sustain said inflammatory reaction during the course of the treatment.

16. Allergen for use according to claim 15, wherein the allergen is in dry form.

17. Allergen for use according to claim 15 or 16, wherein the allergen is applied on intact skin of the subject.

18. Allergen for use according to claim 15 or 16, wherein the allergen is applied after abrasive treatment of the skin.
